# EUROPEAN PATENT APPLICATION

(11) **EP 4 729 572 A1**
(43) Date of publication of application: **22.04.2026**
(21) Application number: 25208458.7
(22) Date of filing: 14.10.2025
(51) Int. Cl.: C08K 5/1545, C08L 5/16, D01D 1/02, D01F 1/10, D01F 2/06, C08G 12/30, C08L 1/02

(54) **VISCOSE-BASED BIOFIBER CONTAINING ACTIVE ANTIOXIDANT COMPONENT AND PREPARATION METHOD THEREOF**

(30) Priority: 15.10.2024 CN 202411435694
(71) Applicant: BYHERB FUTURE HEALTH TECHNOLOGY (QINGDAO) CO., LTD., Qingdao, Shandong 266071 (CN); Byherb Fiber Inc., Fairfax, VA 22033 (US)
(72) Inventor: HUANG, Xiaohua, Qingdao, Shandong, 266071 (CN); HUANG, Yuanlong, Qingdao, Shandong, 266071 (CN)
(74) Representative: Lohr, Jöstingmeier & Partner Patent- und Rechtsanwälte mbB

(57) **Abstract**

The present disclosure discloses a viscose-based biofiber containing an active antioxidant component. The viscose-based biofiber is prepared by reacting following raw materials in parts by weight: 20-24 parts of a Covalent Organic Frameworks (COFs) carrier, 5-8 parts of a plant-derived active antioxidant component, 200-240 parts of a spinning stock solution, 2-4 parts of sodium dodecyl sulfate, 2-4 parts of glyceryl monooleate, 3-5 parts of cyclodextrin, 2-4 parts of titanium dioxide, and 3-5 parts of a silane coupling agent.

## Description

### CROSS-REFERENCES TO RELATED APPLICATIONS

The present disclosure claims priority to Chinese Patent Application No. 202411435694.1, filed on October 15, 2024, the content of which is incorporated herein by reference in its entirety.

### TECHNICAL FIELD

The present disclosure relates to the field of viscose-based biofiber, and more specifically, relates to a viscose-based biofiber containing an active antioxidant component and a preparation method thereof.

### BACKGROUND

Viscose fiber, also known as rayon, is an artificial fiber made from natural fibers such as wood fiber and cotton linters. Viscose fiber is produced by subjecting these raw materials to alkalization, aging, and sulfonation processes to form soluble cellulose xanthate, and the soluble cellulose xanthate is then dissolved in a dilute alkaline solution to prepare viscose. The viscose is used as a spinning solution in a wet spinning process to produce regenerated cellulose fiber. Viscose fiber exhibits excellent moisture absorption, breathability, comfort, smoothness, coolness, antistatic properties, dyeability, and spinnability, making it widely used in clothing, home textiles, and nonwoven fabrics.

Viscose-based biofiber, also known as macro biofunctional viscose fiber, is a viscose polymer with a certain amount of functional materials, which may act on the human body or other organisms. After undergoing special technical processing, the viscose fiber is imparted with one or more functionalities. In recent years, a variety of biofunctional fibers have been continuously developed, both domestically and internationally, providing significant growth opportunities for functional products. The market demand for these fibers has been increasing, and they exhibit broad application prospects in fields such as construction, transportation, water conservation, agriculture, fishery, healthcare, as well as in electronics, communications, and national defense. As a result, functional viscose fiber has become a key development focus in the field of viscose fibers. Due to their unique properties, differentiation, and targeted functionality, functional fiber materials have attracted industry attention and gained market popularity.

The addition of active antioxidant components may enhance the antioxidant properties of viscose-based biofiber. Among them, plant-derived active antioxidant components are highly favored by users due to their excellent environmental compatibility. The primary sources of plant-derived active antioxidant components include tea, mint, lavender, tomato, orange, yucca, Japanese knotweed, and grape.

To improve the antioxidant properties of viscose-based biofiber, there are primarily two modification approaches. The first approach involves blending the active ingredients directly into the spinning solution before fiber spinning. However, when the active ingredient is small in volume, it may be entirely encapsulated within the fiber during spinning, making it difficult for the active ingredient to be released, thereby limiting its effectiveness. The second approach involves first producing the fiber and then attaching the active ingredients to the fiber surface. While this method allows for better release performance of the active ingredient, the active ingredient is prone to detachment after washing, leading to poor wash durability.

To develop a viscose-based biofiber with both excellent antioxidant performance and good wash durability, the present disclosure provides a viscose-based biofiber containing an active antioxidant component and a preparation method thereof.

### SUMMARY

One aspect of the present disclosure provides a viscose-based biofiber containing an active antioxidant component. The viscose-based biofiber is prepared by reacting following raw materials in parts by weight: 20-24 parts of a Covalent Organic Frameworks (COFs) carrier, 5-8 parts of a plant-derived active antioxidant component, 200-240 parts of a spinning stock solution, 2-4 parts of sodium dodecyl sulfate, 2-4 parts of glyceryl monooleate, 3-5 parts of cyclodextrin, 2-4 parts of titanium dioxide, and 3-5 parts of a silane coupling agent.

Another aspect of the present disclosure provides a method for preparing a viscose-based biofiber containing an active antioxidant component. The method includes modifying a COFs carrier, preparing a spinning solution, and spinning. The modifying the COFs carrier includes: mixing 2-4 parts of titanium dioxide with 3-5 parts of a silane coupling agent thoroughly to obtain a first mixture; adding the first mixture to an ethanol solution including the COFs carrier, where an amount of the COFs carrier is 20-24 parts, and a mass ratio of the COFs carrier to ethanol is 1:(5-10); adding 5-8 parts of a plant-derived active antioxidant component, 2-4 parts of sodium dodecyl sulfate, 2-4 parts of glyceryl monooleate, and 3-5 parts of cyclodextrin, under stirring conditions, to obtain a second mixture; and heating the second mixture to 65-75 °C, stirring under reflux for 5-8 hours, and subsequently evaporating ethanol to obtain a modified COFs carrier. The preparing the spinning solution includes: fully mixing the modified COFs carrier with a spinning stock solution to obtain the spinning solution. The spinning includes: performing a spinning process using the spinning solution to produce the viscose-based biofiber containing the active antioxidant component.

### DETAILED DESCRIPTION

The following provides a further explanation of the present disclosure in conjunction with specific embodiments.

In the embodiments described below, the experimental methods or testing methods, unless otherwise specified, are conventional methods; and the reagents and materials, unless otherwise specified, are obtained through conventional commercial channels or prepared using conventional methods. Specifically, α-cellulose is purchased from Shanghai Moxian Industrial Co., Ltd.; nano titanium dioxide is purchased from Jiangsu Caiwei Biotechnology Co., Ltd.; cyclodextrin is purchased from Shandong Tongwang Biotechnology Co., Ltd.; sodium dodecyl sulfate and glyceryl monooleate are purchased from Jiangsu Pulesi Biotechnology Co., Ltd.; and the silane coupling agent is KH560, purchased from Jiangsu JiuJia Biotechnology Co., Ltd.

In the embodiments described below, the preparation method of the Covalent Organic Frameworks (COFs) carrier includes the following steps:

108.6 mg of 2,4,6-tris(4-aminophenyl)-1,3,5-triazine and 105 mg of 2,3,5,6-tetrafluoroterephthalaldehyde are mixed in a glass tube, followed by adding 2 mL of 1,2-dichlorobenzene and 4 mL of n-butanol. The mixed solution undergoes ultrasonic treatment for 20 min, and then 1 mL of a 6 M acetic acid aqueous solution is added to obtain a uniformly dispersed yellow solution. The yellow solution undergoes nitrogen-freezing-degassing treatment five times to remove oxygen, followed by drying in an oven at 120 °C for 3 days. The final product is centrifugally washed with tetrahydrofuran, acetone, and methanol, filtered with n-hexane, and placed in a vacuum drying oven at 60 °C for vacuum drying for 12 h, resulting in orange-yellow solid powder, which is the desired COFs carrier.

### Embodiment 1

A viscose-based biofiber containing active antioxidant components is prepared by reacting the following raw materials in parts by weight: 20 parts of COFs carrier, 5 parts of plant-derived active antioxidant components, 200 parts of spinning stock solution, 2 parts of sodium dodecyl sulfate, 2 parts of glyceryl monooleate, 3 parts of cyclodextrin, 2 parts of titanium dioxide, and 3 parts of silane coupling agent.

The preparation method of the plant-derived active antioxidant components includes the following steps. Tea leaves are wetted with deionized water and then subjected to freeze-drying treatment. Grapes are washed with deionized water and squeezed into grape juice. The freeze-dried tea leaves are soaked in the grape juice at 0 °C for 10 h. After soaking, ultrasonic treatment is performed for 15 min, followed by a first filtration to obtain a first filtrate. The obtained first filtrate undergoes a first evaporation crystallization to obtain a solid mixture. The solid mixture is pulverized into powder, and the powder is then added to anhydrous ethanol. After ultrasonic treatment for 15 min, a second filtration is performed to obtain a second filtrate, and the second filtrate undergoes a second evaporation crystallization to obtain a plant-derived active antioxidant component.

The tea leaves and the grapes are in a mass ratio of 1:15.

During the preparation of the plant-derived active antioxidant components, seeded grapes are selected, and when the grapes are washed with deionized water and squeezed into grape juice, the grape skins, grape pulp, and grape seeds are all crushed together.

During the preparation of the plant-derived active antioxidant components, the ultrasonic treatment has a frequency of 22 kHz, and a power of 400 W.

During the preparation of the plant-derived active antioxidant components, green tea is selected as tea.

The spinning stock solution includes 8.1% of α-cellulose and 5.5% of sodium hydroxide; and the spinning stock solution has a viscosity of 45 s.

The nano titanium dioxide has a particle size of 10-20 nm.

A method for preparing a viscose-based biofiber containing active antioxidant components includes the following steps:

Step 1: Modification of the COFs carrier. 2 parts of titanium dioxide and 3 parts of a silane coupling agent are thoroughly mixed, and then added to an ethanol solution including the COFs carrier, where the amount of the COFs carrier is 20 parts, and a mass ratio of the COFs carrier to ethanol is 1:5. Under stirring conditions, 5 parts of plant-derived active antioxidant components, 2 parts of sodium dodecyl sulfate, 2 parts of glyceryl monooleate, and 3 parts of cyclodextrin are added. The mixture is stirred and heated to 65 °C, followed by reflux stirring for 5 h. Ethanol is then evaporated to obtain the modified COFs.

Step 2: Preparation of spinning solution. The modified COFs and the spinning stock solution are fully mixed to obtain the spinning solution.

Step 3: Spinning. The spinning solution is used for spinning to produce the viscose-based biofiber containing active antioxidant components.

In Step 3, the spinning solution is poured into a spinning machine at a spinning speed of 15 m/min. The spinning process is carried out in a coagulation bath at 30 °C. After solidification, the fiber undergoes desulfurization, washing, oiling, and drying to obtain the viscose-based biofiber containing active antioxidant components.

In Step 3, the composition of the coagulation bath includes: 115 g/L sulfuric acid, 220 g/L sodium sulfate, and 12 g/L zinc sulfate.

### Embodiment 2

A viscose-based biofiber containing active antioxidant components is prepared by reacting the following raw materials in parts by weight: 24 parts of COFs carrier, 8 parts of plant-derived active antioxidant components, 240 parts of spinning stock solution, 4 parts of sodium dodecyl sulfate, 4 parts of glyceryl monooleate, 5 parts of cyclodextrin, 4 parts of titanium dioxide, and 5 parts of silane coupling agent.

The preparation method of the plant-derived active antioxidant components includes the following steps. Tea leaves are wetted with deionized water and then subjected to freeze-drying treatment. Meanwhile, grapes are washed with deionized water and squeezed into grape juice. The freeze-dried tea leaves are soaked in the grape juice at 5 °C for 12 h. After soaking, ultrasonic treatment is performed for 30 min, followed by a first filtration. The obtained filtrate undergoes a first evaporation crystallization to obtain a solid mixture. The solid mixture is pulverized into powder, which is then added to anhydrous ethanol. After ultrasonic treatment for 30 min, a second filtration is performed, and the collected filtrate undergoes a second evaporation crystallization to obtain the plant-derived active antioxidant components.

The tea leaves and the grapes are in a mass ratio of 1:20.

During the preparation of the plant-derived active antioxidant components, seeded grapes are selected, and when the grapes are washed with deionized water and squeezed into grape juice, the grape skins, grape pulp, and grape seeds are all crushed together.

During the preparation of the plant-derived active antioxidant components, the ultrasonic treatment has a frequency of 24 kHz, and a power of 500 W.

During the preparation of the plant-derived active antioxidant components, white tea is selected as tea.

The spinning stock solution includes 8.3% of α-cellulose and 6% of sodium hydroxide; and the spinning stock solution has a viscosity of 55 s.

The nano titanium dioxide has a particle size of 10-20 nm.

A method for preparing a viscose-based biofiber containing active antioxidant components includes the following steps:

Step 1: Modification of the COFs carrier. 4 parts of titanium dioxide and 5 parts of a silane coupling agent are thoroughly mixed and then added to an ethanol solution including the COFs carrier, where the amount of the COFs carrier is 24 parts and a mass ratio of the COFs carrier to ethanol is 1:10. Under stirring conditions, 8 parts of plant-derived active antioxidant components, 4 parts of sodium dodecyl sulfate, 4 parts of glyceryl monooleate, and 5 parts of cyclodextrin are added. The mixture is stirred and heated to 75 °C, followed by reflux stirring for 8 h. Ethanol is then evaporated to obtain the modified COFs.

Step 2: Preparation of spinning solution. The modified COFs and the spinning stock solution are fully mixed to obtain the spinning solution.

Step 3: Spinning. The spinning solution is used for spinning to produce the viscose-based biofiber containing active antioxidant components.

In Step 3, the spinning solution is poured into a spinning machine at a spinning speed of 18 m/min. The spinning process is carried out in a coagulation bath at 35 °C. After solidification, the fiber undergoes desulfurization, washing, oiling, and drying to obtain the viscose-based biofiber containing active antioxidant components.

In Step 3, the composition of the coagulation bath includes: 120 g/L sulfuric acid, 240 g/L sodium sulfate, and 15 g/L zinc sulfate.

### Embodiment 3

A viscose-based biofiber containing active antioxidant components is prepared by reacting the following raw materials in parts by weight: 22 parts of COFs carrier, 6 parts of plant-derived active antioxidant components, 220 parts of spinning stock solution, 3 parts of sodium dodecyl sulfate, 3 parts of glyceryl monooleate, 4 parts of cyclodextrin, 3 parts of titanium dioxide, and 4 parts of silane coupling agent.

The preparation method of the plant-derived active antioxidant components includes the following steps. Tea leaves are wetted with deionized water and then subjected to freeze-drying treatment. Meanwhile, grapes are washed with deionized water and squeezed into grape juice. The freeze-dried tea leaves are soaked in the grape juice at 3 °C for 11 h. After soaking, ultrasonic treatment is performed for 18 min, followed by a first filtration. The obtained filtrate undergoes a first evaporation crystallization to obtain a solid mixture. The solid mixture is pulverized into powder, and the powder is then added to anhydrous ethanol. After ultrasonic treatment for 18 min, a second filtration is performed, and the collected filtrate undergoes a second evaporation crystallization to obtain the plant-derived active antioxidant components.

The tea leaves and the grapes are in a mass ratio of 1:16.

During the preparation of the plant-derived active antioxidant components, seeded grapes are selected, and when the grapes are washed with deionized water and squeezed into grape juice, the grape skins, grape pulp and grape seeds are all crushed together.

During the preparation of the plant-derived active antioxidant components, the ultrasonic treatment has a frequency of 23 kHz, and a power of 450 W.

During the preparation of the plant-derived active antioxidant components, green tea is selected as tea.

The spinning stock solution includes 8.2% of α-cellulose and 5.7% of sodium hydroxide; and the spinning stock solution has a viscosity of 50 s.

The nano titanium dioxide has a particle size of 10-20 nm.

A method for preparing a viscose-based biofiber containing active antioxidant components includes the following steps:

Step 1: Modification of the COFs carrier. 2-4 parts of titanium dioxide and 3-5 parts of a silane coupling agent are thoroughly mixed and then added to an ethanol solution including a COFs carrier, where the amount of the COFs carrier is 22 parts and a mass ratio of the COFs carrier to ethanol is 1:6. Under stirring conditions, 6 parts of plant-derived active antioxidant components, 3 parts of sodium dodecyl sulfate, 3 parts of glyceryl monooleate, and 4 parts of cyclodextrin are added. The mixture is stirred and heated to 70 °C, followed by reflux stirring for 6 h. Ethanol is then evaporated to obtain the modified COFs.

Step 2: Preparation of spinning solution. The modified COFs and the spinning stock solution are fully mixed to obtain the spinning solution.

Step 3: Spinning. The spinning solution is used for spinning treatment to produce the viscose-based biofiber containing active antioxidant components.

In Step 3, the spinning solution is poured into a spinning machine at a spinning speed of 16 m/min. The spinning process is carried out in a coagulation bath at 32 °C. After solidification, the fiber undergoes desulfurization, washing, oiling, and drying to obtain the viscose-based biofiber containing active antioxidant components.

In Step 3, the composition of the coagulation bath includes: 118 g/L sulfuric acid, 230 g/L sodium sulfate, and 13 g/L zinc sulfate.

### Comparative Example 1

Compared with Embodiment 3, the raw materials for preparing the viscose-based biofiber containing active antioxidant components do not include plant-derived active antioxidant components, while the other aspects are the same as those in Embodiment 3.

### Comparative Example 2

Compared with Embodiment 3, the raw materials for preparing the viscose-based biofiber containing active antioxidant components do not include the COFs carrier, while the other aspects are the same as those in Embodiment 3.

### Comparative Example 3

Compared with Embodiment 3, the raw materials for preparing the viscose-based biofiber containing active antioxidant components do not include titanium dioxide and the silane coupling agent, while the other aspects are the same as those in Embodiment 3.

### Comparative Example 4

Compared with Embodiment 3, the preparation method of the plant-derived active antioxidant components is different. The preparation method of the plant-derived active antioxidant components includes the following steps. Tea leaves are wetted with deionized water and then subjected to freeze-drying treatment. The freeze-dried tea leaves are then soaked in deionized water at 3 °C for 11 h. Meanwhile, grapes are washed with deionized water and squeezed into grape juice. The grape juice is mixed with the tea leaf soaking solution. After mixing, ultrasonic treatment is performed for 18 min, followed by a first filtration. The obtained filtrate undergoes a first evaporation crystallization to obtain a solid mixture. The solid mixture is pulverized into powder, which is then added to anhydrous ethanol. After ultrasonic treatment for 18 min, a second filtration is performed, and the collected filtrate undergoes a second evaporation crystallization to obtain the plant-derived active antioxidant components.

The tea leaves and the grapes are in a mass ratio of 1:16. The other aspects are the same as those in Embodiment 3.

### Performance Testing of Embodiments 1-3 and Comparative Examples 1-4

Performance testing is conducted on Embodiments 1-3 and Comparative Examples 1-4. The testing includes a DPPH radical scavenging experiment and an ABTS⁺ radical scavenging experiment.

### 1. DPPH Radical Scavenging Experiment

A 100×10⁻⁶ M DPPH solution is prepared, and 10 mL of the solution is taken as one sample. 1 g of the viscose-based biofiber prepared in Embodiments 1-3 and Comparative Examples 1-4 is added to each sample. The ultraviolet absorption at 519 nm is recorded. After reacting under dark conditions for 30 min, the absorbance of the mixture is measured, and the DPPH radical scavenging rate is calculated.

### 2. ABTS⁺ Radical Scavenging Experiment

A 100×10⁻⁶ M ABTS⁺ solution is prepared, with ABTS diammonium salt as the solute. 10 mL of the solution is taken as one sample. 1 g of the viscose-based biofiber prepared in Embodiments 1-3 and Comparative Examples 1-4 is added to each sample. The ultraviolet absorption at 734 nm is recorded. After reacting under dark conditions for 30 min, the absorbance of the mixture is measured, and the ABTS radical scavenging rate is calculated.

The measurement results are shown in Table 1.

**Table 1**

| | DPPH Radical Scavenging | | ABTS⁺ Radical Scavenging | |
|---|---|---|---|---|
| | Radical Scavenging Rate Before Washing (%) | Radical Scavenging Rate After 100 Washes (%) | Radical Scavenging Rate Before Washing (%) | Radical Scavenging Rate After 100 Washes (%) |
| Embodiment 1 | 68.5 | 58.6 | 74.6 | 64.4 |
| Embodiment 2 | 67.4 | 59.3 | 73.8 | 64.1 |
| Embodiment 3 | 67.9 | 59.1 | 75.3 | 63.9 |
| Comparative Example 1 | 26.2 | 21.3 | 24.3 | 20.9 |
| Comparative Example 2 | 32.4 | 31.3 | 33.9 | 30.2 |
| Comparative Example 3 | 64.5 | 60.9 | 66.9 | 62.7 |
| Comparative Example 4 | 63.2 | 58.8 | 70.3 | 76.2 |

As shown in Table 1, the viscose-based biofiber prepared in Embodiments 1-3 exhibits excellent antioxidant properties, with a high radical scavenging rate. Moreover, after being washed a plurality of times, the radical scavenging rate exhibits minimal variation, demonstrating high stability. In Comparative Example 1, where the plant-derived active antioxidant components are not included, the radical scavenging rate shows a significant decrease. In Comparative Example 2, where the COFs carrier is not included, the transfer of active components is not sufficiently facilitated, leading to a noticeable decrease in the radical scavenging rate. However, it is slightly superior to Comparative Example 1, and after being washed a plurality of times, the radical scavenging rate remains almost unchanged. In Comparative Example 3, where titanium dioxide and the silane coupling agent are not added, the radical scavenging rate exhibits a slight decrease. In Comparative Example 4, where the preparation method of the plant-derived active antioxidant components differs, the radical scavenging rate shows a slight decrease.

The beneficial effects of the present disclosure are as follows:
1. The viscose-based biofiber prepared in the present disclosure includes active antioxidant components attached to the COFs carrier during the preparation process. In the prepared viscose-based biofiber, the COFs carrier serves as the structural framework of the viscose-based biofiber, with the ends of the COFs carrier being exposed or positioned near the surface of the viscose-based biofiber. This facilitates the transfer of the active components from the COFs carrier to the surface of the viscose-based biofiber, ensuring that the active components effectively exert their function, thereby achieving excellent antioxidant performance.
2. The viscose-based biofiber prepared in the present disclosure includes plant-derived active antioxidant components during the preparation process, effectively enhancing the environmental performance of the viscose-based biofiber. During the preparation of the plant-derived active antioxidant components, the active components in tea leaves and grapes are effectively retained, improving the antioxidant performance of the viscose-based biofiber.
3. The viscose-based biofiber prepared in the present disclosure includes titanium dioxide and a silane coupling agent during the preparation process. The titanium dioxide encapsulated by the silane coupling agent exhibits a certain degree of photocatalytic activity, which may enhance the antioxidant performance of the viscose-based biofiber.

The above descriptions represent only preferred specific embodiments of the present disclosure. However, the scope of protection of the present disclosure is not limited thereto. Any equivalent substitutions or modifications made by those skilled in the art within the technical scope disclosed in the present disclosure, based on the technical solutions and inventive concepts of the present disclosure, shall fall within the scope of protection of the present disclosure.

## Claims

1. A viscose-based biofiber comprising an active antioxidant component, wherein the viscose-based biofiber is prepared by reacting following raw materials in parts by weight: 20-24 parts of a Covalent Organic Frameworks (COFs) carrier, 5-8 parts of a plant-derived active antioxidant component, 200-240 parts of a spinning stock solution, 2-4 parts of sodium dodecyl sulfate, 2-4 parts of glyceryl monooleate, 3-5 parts of cyclodextrin, 2-4 parts of titanium dioxide, and 3-5 parts of a silane coupling agent.

2. The viscose-based biofiber according to claim 1, wherein the plant-derived active antioxidant component is prepared by following:
wetting tea leaves with deionized water, followed by freeze-drying treatment;
washing grapes with deionized water and squeezing the grapes into grape juice;
soaking freeze-dried tea leaves in the grape juice at 0-5 °C for 10-12 hours;
performing ultrasonic treatment for 15-30 minutes after soaking;
conducting a first filtration to obtain a first filtrate;
subjecting the first filtrate to a first evaporation crystallization to obtain a solid mixture;
pulverizing the solid mixture into powder;
adding the powder into anhydrous ethanol and performing ultrasonic treatment for 15-30 minutes;
conducting a second filtration to collect a second filtrate; and
subjecting the second filtrate to a second evaporation crystallization to obtain the plant-derived active antioxidant component,
wherein the tea leaves and the grapes are in a mass ratio of 1:(15-20).

3. The viscose-based biofiber according to claim 2, wherein the grapes comprise seeded grapes; and the grape juice is prepared by crushing grape skins, grape pulp, and grape seeds together.

4. The viscose-based biofiber according to claim 2, wherein the ultrasonic treatment has a frequency of 22-24 kHz, and a power of 400-500 W.

5. The viscose-based biofiber according to claim 2, wherein the tea leaves comprise unfermented tea leaves or lightly fermented tea leaves.

6. The viscose-based biofiber according to claim 1, wherein
the spinning stock solution comprises 8.1%-8.3% of α-cellulose and 5.5%-6% of sodium hydroxide; and
the spinning stock solution has a viscosity of 45-55 s.

7. The viscose-based biofiber according to claim 1, wherein the titanium dioxide has a particle size of 10-20 nm.

8. A method, for preparing a viscose-based biofiber comprising an active antioxidant component, comprising: modifying a COFs carrier, preparing a spinning solution, and spinning, wherein
modifying the COFs carrier comprises:
mixing titanium dioxide with a silane coupling agent thoroughly to obtain a first mixture;
adding the first mixture to an ethanol solution comprising the COFs carrier;
adding a plant-derived active antioxidant component, sodium dodecyl sulfate, glyceryl monooleate, and cyclodextrin, to obtain a second mixture; and
heating the second mixture and evaporating ethanol to obtain a modified COFs carrier;
preparing the spinning solution comprises: fully mixing the modified COFs carrier with a spinning stock solution to obtain the spinning solution; and
the spinning comprises: performing a spinning process using the spinning solution to produce the viscose-based biofiber comprising the active antioxidant component, and
wherein the titanium dioxide may have a particle size of 10-20 nm..

9. The method according to claim 8, further comprising:
pouring the spinning solution into a spinning machine, wherein the spinning is performed at a speed of 15-18 m/min, and the spinning process takes place in a coagulation bath at 30-35 °C; and
after solidification, performing desulfurization, washing, oiling, and drying to obtain the viscose-based biofiber comprising the active antioxidant component.

10. The method according to claim 9, further comprising: preparing the coagulation bath by using 115-120 g/L of sulfuric acid, 220-240 g/L of sodium sulfate, and 12-15 g/L of zinc sulfate.

11. The method according to claim 8, further comprising: preparing the plant-derived active antioxidant component,
wherein the preparing the plant-derived active antioxidant component comprises:
wetting tea leaves with deionized water, followed by freeze-drying treatment;
washing grapes with deionized water and squeezing the grapes into grape juice;
soaking the freeze-dried tea leaves in the grape juice at 0-5 °C for 10-12 hours;
performing ultrasonic treatment for 15-30 minutes after soaking;
conducting a first filtration to obtain a first filtrate;
subjecting the first filtrate to a first evaporation crystallization to obtain a solid mixture;
pulverizing the solid mixture into powder;
adding the powder into anhydrous ethanol and performing ultrasonic treatment for 15-30 minutes;
conducting a second filtration to collect a second filtrate; and
subjecting the second filtrate to a second evaporation crystallization to obtain the plant-derived active antioxidant component,
wherein the tea leaves and the grapes are in a mass ratio of 1:(15-20).

12. The method according to claim 11, further comprising: preparing the grape juice by crushing grape skins, grape pulp, and grape seeds together, wherein the grapes comprises seeded grapes.

13. The method according to claim 11, wherein performing the ultrasonic treatment comprises: performing the ultrasonic treatment using a frequency of 22-24 kHz and a power of 400-500 W.

14. The method according to claim 8, wherein modifying the COFs carrier further comprises:
mixing 2-4 parts of titanium dioxide with 3-5 parts of the silane coupling agent thoroughly to obtain the first mixture, wherein, when adding the first mixture to the ethanol solution comprising the COFs carrier, an amount of the COFs carrier is 20-24 parts, and the mass ratio of the COFs carrier to the ethanol is 1:(5-10);
adding 5-8 parts of the plant-derived active antioxidant component, 2-4 parts of the sodium dodecyl sulfate, 2-4 parts of the glyceryl monooleate, and 3-5 parts of the cyclodextrin, under stirring conditions, to obtain the second mixture; and
heating the second mixture to 65-75 °C, stirring under reflux for 5-8 hours, and subsequently evaporating the ethanol to obtain the modified COFs carrier.

15. The method according to claim 8, further comprising:
preparing the spinning stock solution by using 8.1%-8.3% of α-cellulose and 5.5%-6% of sodium hydroxide,
wherein the spinning stock solution has a viscosity of 45-55 s.
